(19) 

(11) **EP 4 789 690 A1**

(12) **EUROPEAN PATENT APPLICATION**
published in accordance with Art. 153(4) EPC

(43) Date of publication:
**12.08.2026 Bulletin 2026/33**

(21) Application number: **24874485.6**

(22) Date of filing: **19.09.2024**

(51) International Patent Classification (IPC):
***A61L 9/014*** *(2006.01)* ***A61L 9/01*** *(2006.01)*
***B01J 20/22*** *(2006.01)* ***C08G 77/26*** *(2006.01)*

(52) Cooperative Patent Classification (CPC):
**A61L 9/01; A61L 9/014; B01D 53/14; B01J 20/22; B01J 20/28; C08G 77/26**

(86) International application number:
**PCT/JP2024/033478**

(87) International publication number:
**WO 2025/074876 (10.04.2025 Gazette 2025/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **02.10.2023 JP 2023171201**

(71) Applicant: **Tosoh Corporation**
**Shunan-shi, Yamaguchi 746-8501 (JP)**

(72) Inventors:
- **KISANUKI, Sayaka**
  **Shunan-shi, Yamaguchi 746-8501 (JP)**
- **SUDOU, Yukinori**
  **Shunan-shi, Yamaguchi 746-8501 (JP)**
- **YAMAGUCHI, Kenta**
  **Shunan-shi, Yamaguchi 746-8501 (JP)**

(74) Representative: **Vossius & Partner**
**Patentanwälte Rechtsanwälte mbB**
**Siebertstraße 3**
**81675 München (DE)**

# (54) DEODORANT COMPOSITION AND DEODORIZING FILTER

(57) Provided is a deodorant composition comprising activated carbon with excellent removal rate and storage stability.

The deodorant composition comprises activated carbon and an aminooxyalkyl-containing polysiloxane having a structural unit represented by the following formula (1) which is loaded on the activated carbon.

(1)

[In the formula, each R is independently a $C_{1-4}$ alkyl group which may be substituted with a primary amino group at any position, a $C_{1-4}$ alkoxy group or a hydroxy group, each Q is independently at least one group selected from the group consisting of $-CH_2-$, $-CH_2CH_2-$ and $-CH_2-Ra-CH_2-$ wherein Ra is a $C_{1-18}$ alkylene group in which two or more interspaced $-CH_2-$ groups may each be independently replaced by -O-, -(C=O)O-, - O(C=O)-, -O(C=O)-O-, -C(=O)-NH-, -NH-(C=O)-, -CH=CH- or -C=C-, and in which a hydrogen atom in the alkylene group may be substituted with a $C_{1-3}$ alkyl group, and n is a real number of from 0 to 100.]

EP 4 789 690 A1

Fig. 1

Intens.
+MS, 2.1-2.6min #126-156, Background Subtracted
1243.9654
659.2856
815.1861
1543.9495
0
250
500
750
1000
1250
500
750
1000
1250
1500
1750
2000
2250
2500
2750
m/z

## Description

TECHNICAL FIELD

The present invention relates to a deodorant composition and a deodorizing filter.

BACKGROUND ART

Deodorant compositions and deodorizing filters containing activated carbon have been used conventionally to eliminate malodors.

Activated carbon removes odors through adsorption of odorous molecules, but its adsorption capacity is not infinite. Once its adsorption reaches saturation, desorption starts, and adsorbed odorous molecules are released from activated carbon. In addition, activated carbon alone is not sufficient to remove malodorous gases, especially aldehyde gases.

Therefore, removal of malodorous gases using activated carbon loaded with an aldehyde scavenger which can capture aldehydes through chemical reaction has been studied. As aldehyde scavengers, hydrazine derivatives, amines, amino acids or urea derivatives are known (Patent Documents 1 to 4).

PRIOR ART DOCUMENTS

PATENT DOCUMENTS

| | |
|---|---|
| Patent Document 1: | JP-A-H11-299878 |
| Patent Document 2: | JP-A-2000-300652 |
| Patent Document 3: | JP-A-2007-215818 |
| Patent Document 4: | JP-A-2018-108360 |

DISCLOSURE OF INVENTION

TECHNICAL PROBLEM

However, activated carbon loaded with an aldehyde scavenger disclosed in these patent documents has problems since it cannot remove aldehydes efficiently enough and its performance lowers rapidly over time.

Considering the above-mentioned background art, the present invention aims to provide a deodorant composition which can remove aldehydes efficiently even after long storage in air.

SOLUTION TO PROBLEM

As a result of their extensive research to solve the above-mentioned problems, the present inventors found a composition comprising activated carbon loaded with the particular polysiloxane described later solves the above-mentioned problems and accomplished the present invention.

Namely, the following embodiments are disclosed herein.

[1] A deodorant composition comprising activated carbon and an aminooxyalkyl-containing polysiloxane having a structural unit represented by the following formula (1) which is loaded on the activated carbon.

(1)

[wherein each R is independently a $C_{1-4}$ alkyl group which may be substituted with a primary amino group at any position, a $C_{1-4}$ alkoxy group or a hydroxy group, each Q is independently at least one group selected from the group consisting of $-CH_2-$, $-CH_2CH_2-$and $-CH_2-Ra-CH_2-$ wherein Ra is a $C_{1-18}$ alkylene group in which two or more interspaced $-CH_2-$ groups may each be independently replaced by -O-, -(C=O)O-, - O(C=O)-, -O(C=O)-O-, -C(=O)-NH-, -NH-(C=O)-, -CH=CH- or -C=C-, and in which a hydrogen atom in the alkylene group may be substituted with a $C_{1-3}$ alkyl group, and n is a real number of from 0 to 100.]

[2] The deodorant composition according to [1], wherein in the aminooxyalkyl-containing polysiloxane represented by the formula (1), each R is independently a methyl group, a 3-aminopropyl group, a methoxy group, an ethoxy group, a propyloxy group, an isopropyloxy group or a hydroxy group.

[3] The deodorant composition according to [1] or [2], wherein in the aminooxyalkyl-containing polysiloxane represented by the formula (1), n is 0.

[4] The deodorant composition according to any one of [1] to [3], wherein the amount of the aminooxyalkyl-containing polysiloxane represented by the formula (1) is from 0.1 to 100 wt% of the activated carbon.

[5] A deodorizing filter comprising the deodorant composition as defined in any one of [1] to [4].

[6] The deodorizing filter according to [5], wherein the content of the deodorant composition is from 1 to 90 wt% of the deodorizing filter.

[7] A method for removing an aldehyde, which comprises bringing the deodorant composition as defined in any one of [1] to [4] into contact with a gas containing an aldehyde.

[8] A method for removing an aldehyde, which comprises bringing the deodorizing filter as defined in [5] or [6] into contact with a gas containing an aldehyde.

## ADVANTAGEOUS EFFECTS OF INVENTION

The deodorant composition disclosed herein can remove aldehydes quickly and persistently, and as a result, can improve the living environment by decreasing aldehydes harmful to humans.

## DESCRIPTION OF EMBODIMENTS

Now, embodiments of the deodorant composition and the deodorizing filter disclosed herein will be described in detail. Herein, "to" expresses a numerical range including the figures before and after "to" as the lower limit and the upper limit.

Herein, the deodorant composition comprises activated carbon and an aminooxyalkyl-containing polysiloxane having a structural unit represented by the formula (1) which is loaded on the activated carbon.

In the formula (1), each R is independently a $C_{1-4}$ alkyl group which may be substituted with a primary amino group at any position, a $C_{1-4}$ alkoxy group or a hydroxy group.

The $C_{1-4}$ alkyl group is not particularly restricted and may, for example, be a methyl group, an ethyl group, a propyl group, an isopropyl group, a 3-aminopropyl group, a butyl group, a 2-methylpropyl group, a 1-methylpropyl group or a tert-butyl group.

The $C_{1-4}$ alkoxy group is not particularly restricted and may, for example, be a methoxy group, an ethoxy group, a propyloxy group, an isopropyloxy group, a butoxy group, a 2-methylpropyloxy group, a 1-methylpropyloxy group or a tert-butoxy group.

Among them, R is preferably a methyl group, a 3-aminopropyl group, a methoxy group, an ethoxy group, a propyloxy group, an isopropyloxy group or a hydroxy group for more efficient removal of aldehydes.

Each Q is independently at least one group selected from the group consisting of $-CH_2-$, $-CH_2CH_2-$ and $-CH_2-Ra-CH_2-$.

Ra is a $C_{1-18}$ alkylene group in which two or more interspaced $-CH_2-$ groups may each be independently replaced by -O-, -(C=O)O-, -O(C=O)-, -O(C=O)-O-, -C(=O)-NH-, -NH-(C=O)-, -CH=CH- or -C=C-, and in which a hydrogen atom in the alkylene group may be substituted with a $C_{1-3}$ alkyl group.

Q is not particularly restricted, and its specific examples include structures represented by the following chemical formulae (Q-1) to (Q-95).

(Q-1) (Q-2) (Q-3) (Q-4) (Q-5)

(Q-6) (Q-7) (Q-8) (Q-9)

(Q-10) (Q-11) (Q-12)

(Q-13) (Q-14)

(Q-15) (Q-16)

(Q-17) (Q-18)

(Q19) (Q-20) (Q-21) (Q-22) (Q-23) (Q-24) (Q-25)

(Q-26) (Q-27) (Q-28) (Q-29) (Q-30) (Q-31)

(Q-32) (Q-33) (Q-34) (Q-35) (Q-36) (Q-37)

(Q-38) (Q-39) (Q-40) (Q-41) (Q-42) (Q-43)

(Q-44) (Q-45) (Q-46) (Q-47) (Q-48)

(Q-49) (Q-50) (Q-51) (Q-52) (Q-53)

(Q-54) (Q-55) (Q-56) (Q-57) (Q-58)

(Q-59) (Q-60) (Q-61) (Q-62) (Q-63)

(Q-64) (Q-65) (Q-66) (Q-67)

(Q-68) (Q-69) (Q-70) (Q-71)

(Q-72) (Q-72) (Q-73) (Q-74)

(Q-75) (Q-76) (Q-77) (Q-78)

(Q-79) (Q-80) (Q-81) (Q-82)

(Q-83) (Q-84) (Q-85) (Q-86)

(Q-87) (Q-88) (Q-89) (Q-89)

(Q-90) (Q-91) (Q-92) (Q-93)

(Q-94) (Q-95)

[in the chemical formulae (Q-1) to (Q-95), a hydrogen atom in an alkylene group may be substituted with a $C_{1-3}$ alkyl group, and * represents a bond shown in the formula (1).]

The $C_{1-3}$ alkyl group is not particularly restricted and may, for example, be a methyl group, an ethyl group, a propyl group or an isopropyl group.

In the formula (1), n is a real number from 0 to 100 and is preferably from 0 to 10 for more efficient removal of aldehydes.

The amount of the aminooxyalkyl-containing polysiloxane represented by the formula (1) is not particularly restricted and is preferably from 0.1 to 100 wt%, more preferably from 5 to 100 wt%, further preferably from 40 to 100 wt%, relative to the activated carbon.

The molecule of the aminooxy-containing polysiloxane represented by the formula (1) may be in any shape without particular restrictions and may be linear, branched or cyclic.

Herein, the deodorant composition comprises an aminooxyalkyl-containing polysiloxane having a structural unit represented by the formula (1) and activated carbon and may further comprise an acid, a base, a binder, a thickener, an

anti-foaming agent, a surfactant, an anti-fungus agent, an antiseptic agent or the like.

The acid is not particularly restricted and may, for example, be an inorganic acid such as hydrochloric acid, hydrobromic acid, perchloric acid, silicic acid, tetrafluoroboric acid, hexafluorophosphoric acid, sulfuric acid, nitric acid and phosphoric acid, or an organic acid such as acetic acid, citric acid, fumaric acid, maleic acid, trifluoromethanesulfonic acid, trifluoroacetic acid, benzoic acid, p-toluenesulfonic acid, malonic acid, barbituric acid, 1,3-dimethylbarbituric acid and Meldrum's acid. The acid is preferably hydrochloric acid, sulfuric acid, phosphoric acid or barbituric acid for more efficient removal of aldehydes.

The base is not particularly restricted and may, for example, be an inorganic base such as ammonia, lithium hydroxide, sodium hydroxide, potassium hydroxide, rubidium hydroxide, cesium hydroxide, lithium carbonate, sodium carbonate, potassium carbonate, rubidium carbonate, cesium carbonate, lithium hydrogen carbonate, sodium hydrogen carbonate, potassium hydrogen carbonate, rubidium hydrogen carbonate and cesium hydrogen carbonate, or an organic base such as lithium citrate, sodium citrate, potassium citrate, rubidium citrate, cesium citrate, lithium trifluoromethanesulfonate, sodium trifluoromethanesulfonate, potassium trifluoromethanesulfonate, rubidium trifluoromethanesulfonate, cesium trifluoromethanesulfonate, polyethyleneimine, ethanolamine, diethanolamine, triethanolamine, tris(hydroxymethyl)aminomethane, ethylenediamine, diethylenetriamine, triethylenediamine, triethylenetetramine, tetraethylenepentamine, pentaethylenehexamine, ethylenediamine-N,N,N',N'-tetraethanol, 1,4-piperazinediethanol, 1-(2,3-dihydroxypropyl)piperazine, 2-hydroxymethyltriethylenediamine, piperazine and N-(2-aminoethyl)piperazine. The base is preferably ammonia, sodium hydroxide, potassium hydroxide, sodium carbonate, sodium trifluoromethanesulfonate or tris(hydroxymethyl)aminomethane for more efficient removal of aldehydes.

Herein, the content of an acid or a base, if any, in the deodorant agent is not particularly restricted and is preferably from 0.001 to 10 molar equivalents, more preferably from 0.005 to 5 equivalents, more preferably from 0.01 to 2 equivalents, relative to 1 mole of the group represented by $-ONH_2$.

The surfactant is not particularly restricted and may, for example, be an anionic surfactant such as a linear alkylbenzene sulfonate, an alkyl ether sulfate salt and a polyoxyethylene alkyl ether carboxylate, a nonionic surfactant such as a polyoxyethylene alkyl ether, a polyoxyalkylene alkyl ether and a polyoxyalkylene alkylamine, an amphoteric surfactant such as an alkylamino fatty acid salt, an alkylbetaine and an alkylamine oxide or a cationic surfactant such as alkyltrimethylammonium chloride, dialkyldimethylammonium chloride, a dialkylammonium salt called an ester quat or an alkylammonium salt called an amide quat. Among them, preferred is a linear alkylbenzene sulfonate such as sodium decylbenzenesulfonate, sodium undecylbenzenesulfonate, sodium dodecylbenzenesulfonate, sodium tridecylbenzenesulfonate, sodium tetradecylbenzenesulfonate, sodium pentadecylbenzenesulfonate, sodium hexadecylbenzenesulfonate, sodium heptadecylbenzenesulfonate or sodium octadecylbenzenesulfonate.

Herein, the content of a binder, a thickener, an anti-foaming agent, a surfactant, an anti-fungus agent or an antiseptic agent, if any, in the deodorant composition is not particularly restricted and is preferably from 0.01 to 100 wt%, more preferably from 0.01 to 30 wt%, more preferably from 0.01 to 10 wt%, relative to the activated carbon, respectively.

Herein, the deodorant composition comprises an aminooxyalkyl-containing polysiloxane represented by the formula (1) loaded on activated carbon.

Herein, the deodorant composition may be produced by any methods without particular restrictions and may, for example, be produced by applying a solution of the aminooxyalkyl-containing polysiloxane represented by the formula (1) (hereinafter referred to as a polysiloxane solution) onto activated carbon or by immersing activated carbon into a polysiloxane solution.

The solvent in the polysiloxane solution is not particularly restricted and may, for example, be water, methanol, ethanol, toluene or the like. Among them, water is preferred because it is inexpensive and harmless to humans.

After loading of the aminooxyalkyl-containing polysiloxane represented by the formula (1) onto activated carbon, the resulting deodorant composition may be dried, if necessary. The drying conditions can be adjusted arbitrarily in accordance with the intended use without any particular restrictions. For example, the temperature is from 0 to 200°C, and the time is from several minutes to 48 hours.

Herein, the deodorant composition may be incorporated into industrial products.

The industrial products are not particularly restricted and include conventionally known products and materials such as paints, adhesives, ink, sealants, paper products, binders, resin emulsions, pulp, wooden materials, wooden products, plastic products, films, wall papers, building materials (like plasterboard, interior materials, ceiling materials and flooring), textiles, filters like deodorizing filters for air cleaners and cabin air, and composites thereof such as composites of wooden materials and plastic materials.

When the industrial product is a filter (especially, a deodorizing filter), it may have any conventionally known structure.

Herein, it is possible to remove an aldehyde by bringing the deodorant composition into contact with air containing the aldehyde.

Herein, it is possible to remove an aldehyde by bringing the deodorizing filter into contact with air containing the aldehyde.

BRIEF DESCRIPTION OF DRAWINGS

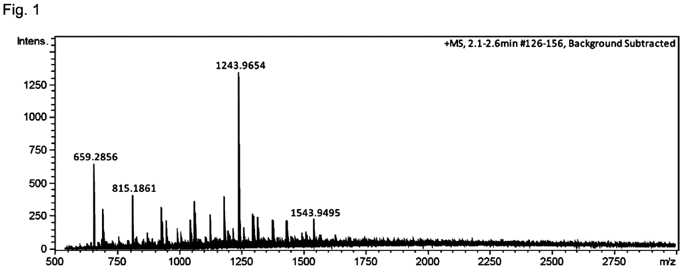

[Fig. 1] The mass spectrum of the aminooxyalkyl-containing polysiloxane obtained in Synthetic Example 9 taken on a mass spectrometer (microTOF, manufactured by Bruker Daltonics) for molecular weight determination.
[Fig. 2] Schematic view of the tester used in the aldehyde removal test in Examples 28 to 29 and Comparative Examples 12 to 13.

EXAMPLES

Now, the above-mentioned embodiments will be described in further detail with reference to Examples. However, it should be understood that these Examples are intended to help understand the present invention, and the present invention is by no means restricted thereto. As the reagents, commercially available products were used unless otherwise noted.

For the compounds used in the Examples, the following abbreviations are used.

O-[3-(Triethoxysilyl)propyl]hydroxylamine: Si-1
3-(Triethoxysilyl)propylamine: Si-2
3-(Diethoxymethylsilyl)propylamine: Si-3
Tetraethoxysilane: TEOS
Triethoxymethylsilane: TEMS
Diethoxydimethylsilane: DEDMS

$(EtO)_2Si(R^1)CH_2CH_2CH_2R^2$ $(EtO)_2Si(R^3)-R^4$

Si-1: $R^1$= OEt, $R^2$= $ONH_2$ TEOS : $R^3$, $R^4$= OEt
Si-2: $R^1$= OEt, $R^2$= $NH_2$ TEMS : $R^3$= OEt, $R^4$= Me
Si-3: $R^1$= Me, $R^2$= $NH_2$ DEDMS: $R^3$, $R^4$= Me

<SYNTHETIC EXAMPLE 1>

A mixture of Si-1 (0.78 g, 3.29 mmol) and water (9.22 g) was refluxed (on an oil bath at 110°C) with stirring for 20 hours to obtain a transparent solution containing an aminooxyalkyl-containing polysiloxane. The molecular weight of the aminooxyalkyl-containing polysiloxane was measured with a gel permeation chromatograph (Prominence, manufactured by Shimadzu Corporation), and it turned out that the number-average molecular weight (Mn) was 930, the weight average molecular weight (Mw) was 1100, and the dispersity (Mw/Mn) was 1.18.

<SYNTHETIC EXAMPLE 2>

From a mixture of Si-1 (2.00 g, 8.43 mmol) and water (8.00 g), a transparent solution containing an aminooxyalkyl-containing polysiloxane was obtained in the same manner as in Synthetic Example 1. The molecular weight of the aminooxyalkyl-containing polysiloxane was measured in the same manner as in Synthetic Example 1, and it turned out that Mn = 940, Mw = 990, and Mw/Mn = 1.05.

<SYNTHETIC EXAMPLE 3>

A mixture of Si-1 (0.78 g, 3.29 mmol) and water (9.22 g) was refluxed (on an oil bath at 110°C) with stirring for 20 hours and was allowed to stand at 60°C for 14 days to obtain a transparent solution containing an aminooxyalkyl-containing polysiloxane. The molecular weight of the aminooxyalkyl-containing polysiloxane was measured in the same manner as in Synthetic Example 1, and it turned out that Mn = 1100, Mw = 1300, and Mw/Mn = 1.18.

<SYNTHETIC EXAMPLE 4>

From a mixture of Si-1 (0.78 g, 3.29 mmol), 1 mol/L hydrochloric acid (3.29 g, 3.29 mmol) and water (5.93 g), a

transparent solution containing an aminooxyalkyl-containing polysiloxane was obtained in the same manner as in Synthetic Example 1. The molecular weight of the aminooxyalkyl-containing polysiloxane was measured in the same manner as in Synthetic Example 1, and it turned out that Mn = 890, Mw = 910, and Mw/Mn = 1.02.

<SYNTHETIC EXAMPLE 5>

From a mixture of Si-1 (0.78 g, 3.29 mmol), 1 mol/L sulfuric acid (0.33 g, 0.33 mmol) and water (8.89 g), a transparent solution containing an aminooxyalkyl-containing polysiloxane was obtained in the same manner as in Synthetic Example 1. The molecular weight of the aminooxyalkyl-containing polysiloxane was measured in the same manner as in Synthetic Example 1, and it turned out that Mn = 1400, Mw = 1700, and Mw/Mn = 1.21.

<SYNTHETIC EXAMPLE 6>

From a mixture of Si-1 (0.78 g, 3.29 mmol), 14.6 mol/L phosphoric acid (0.02 g, 0.33 mmol) and water (9.20 g), a transparent solution containing an aminooxyalkyl-containing polysiloxane was obtained in the same manner as in Synthetic Example 1. The molecular weight of the aminooxyalkyl-containing polysiloxane was measured in the same manner as in Synthetic Example 1, and it turned out that Mn = 1200, Mw = 1200, and Mw/Mn = 1.00.

<SYNTHETIC EXAMPLE 7>

From a mixture of Si-1 (0.78 g, 3.29 mmol), Si-2 (0.08 g, 0.33 mmol) and water (9.15 g), a transparent solution containing an aminooxyalkyl-containing polysiloxane was obtained in the same manner as in Synthetic Example 1. The molecular weight of the aminooxyalkyl-containing polysiloxane was measured in the same manner as in Synthetic Example 1, and it turned out that Mn = 890, Mw = 920, and Mw/Mn = 1.03.

<SYNTHETIC EXAMPLE 8>

From a mixture of Si-1 (0.78 g, 3.29 mmol), Si-3 (0.97 g, 3.29 mmol) and water (8.25 g), a transparent solution containing an aminooxyalkyl-containing polysiloxane was obtained in the same manner as in Synthetic Example 1. The molecular weight of the aminooxyalkyl-containing polysiloxane was measured in the same manner as in Synthetic Example 1, and it turned out that Mn = 1200, Mw = 1600, and Mw/Mn = 1.33.

<SYNTHETIC EXAMPLE 9>

From a mixture of Si-1 (0.78 g, 3.29 mmol), TEOS (0.21 g, 1.01 mmol) and water (9.01 g), a transparent solution containing an aminooxyalkyl-containing polysiloxane was obtained in the same manner as in Synthetic Example 1. The molecular weight of the aminooxyalkyl-containing polysiloxane was measured in the same manner as in Synthetic Example 1, and it turned out that Mn = 1000, Mw = 1200, and Mw/Mn = 1.20.

<SYNTHETIC EXAMPLE 10>

From a mixture of Si-1 (0.78 g, 3.29 mmol), TEMS (1.0 g, 3.29 mmol) and water (8.22 g), a transparent solution containing an aminooxyalkyl-containing polysiloxane was obtained in the same manner as in Synthetic Example 1. The molecular weight of the aminooxyalkyl-containing polysiloxane was measured in the same manner as in Synthetic Example 1, and it turned out that Mn = 2100, Mw = 3900, and Mw/Mn = 1.86.

<SYNTHETIC EXAMPLE 11>

From a mixture of Si-1 (0.78 g, 3.29 mmol), DEDMS (1.3 g, 3.29 mmol) and water (7.92 g), a transparent solution containing an aminooxyalkyl-containing polysiloxane was obtained in the same manner as in Synthetic Example 1. The molecular weight of the aminooxyalkyl-containing polysiloxane was measured in the same manner as in Synthetic Example 1, and it turned out that Mn = 1700, Mw = 2500, and Mw/Mn = 1.47.

<SYNTHETIC EXAMPLE 12>

From a mixture of Si-2 (0.73 g, 3.29 mmol) and water (9.27 g), a transparent solution containing an aminoalkyl-containing polysiloxane was obtained in the same manner as in Synthetic Example 1. The molecular weight of the aminoalkyl-containing polysiloxane was measured in the same manner as in Synthetic Example 1, and it turned out that

Mn = 750, Mw = 770, and Mw/Mn = 1.02.

The raw materials used for preparation of polysiloxane solutions in Synthetic Examples 1 to 12 and the molecular weights of the polysiloxanes prepared are shown in Table 1.

[Table 1]

| | Raw materials* [wt%] | | | Reflux time [h] | Heating at 60°C [days] | Molecular weight | | |
|---|---|---|---|---|---|---|---|---|
| | Si-1 | Silane compound | Additive | | | Mn | Mw | Mw/Mn |
| Synthetic Ex. 1 | 7.8 | - | - | 20 | - | 930 | 1100 | 1.2 |
| Synthetic Ex. 2 | 20 | - | - | 20 | - | 940 | 990 | 1.1 |
| Synthetic Ex. 3 | 7.8 | - | - | 20 | 14 | 1100 | 1300 | 1.2 |
| Synthetic Ex. 4 | 7.8 | - | 1 mol/L hydrochloric acid 32.9 | 20 | - | 890 | 910 | 1.0 |
| Synthetic Ex. 5 | 7.8 | - | 1 mol/L sulfuric acid 3.3 | 20 | - | 1400 | 1700 | 1.2 |
| Synthetic Ex. 6 | 7.8 | - | 14.6 mol/L phosphoric acid 0.2 | 20 | - | 1200 | 1200 | 1.0 |
| Synthetic Ex. 7 | 7.8 | Si-2, 0.8 | - | 20 | - | 890 | 920 | 1.0 |
| Synthetic Ex. 8 | 7.8 | Si-3, 9.7 | - | 20 | - | 1200 | 1600 | 1.3 |
| Synthetic Ex. 9 | 7.8 | TEOS, 2.1 | - | 20 | - | 1000 | 1200 | 1.2 |
| Synthetic Ex. 10 | 7.8 | TEMS, 10 | - | 20 | - | 2100 | 3900 | 1.9 |
| Synthetic Ex. 11 | 7.8 | DEDMS, 13 | - | 20 | - | 1700 | 2500 | 1.5 |
| Synthetic Ex. 12 | - | Si-2, 7.3 | - | 20 | - | 750 | 770 | 1.0 |
| * The balance is water. | | | | | | | | |

EXAMPLE 1

<Preparation of Deodorant Composition>

0.3 g of the polysiloxane solution (hereinafter referred to as the loading solution) prepared in Synthetic Example 1 was added dropwise to 0.3 g of activated carbon (Shirasagi KL, manufactured by Osaka Gas Chemicals Co., Ltd., mesh 10 to 60), and the activated carbon was dried in a hot-air dryer (ADVANTEC DRJ433DA) at 90°C for 3 hours to obtain a deodorant composition comprising an aminooxyalkyl-containing polysiloxane.

<Acetaldehyde Removal Test>

0.25 g of the deodorant composition was sealed in a 10 L Tedlar bag, and 5 L of nitrogen gas containing 1 ppm acetaldehyde was injected. After 2 hours of standing at room temperature, the gas in the Tedlar bag was passed through a 2,4-dinitrophenylhydrazine (DNPH) cartridge column (Precep-C DNPH, manufactured by FUJIFILM Wako Pure Chemical Corporation) to adsorb the aldehyde in the gas on it. The DNPH-aldehyde condensate was eluted from the column with acetonitrile. The eluate was analyzed with a liquid chromatograph (LC-2030C Plus, manufactured by Shimadzu Corporation) to determine the concentration of the acetaldehyde. The acetaldehyde removal rate was calculated from

the following formula.

Acetaldehyde removal rate [%] = [(Initial acetaldehyde concentration - Remaining acetaldehyde concentration) / Initial acetaldehyde concentration] $\times$ 100

EXAMPLE 2

In Example 2, a deodorant composition was prepared in the same manner as in Example 1 except that the activated carbon was changed to Shirasagi G2x, manufactured by Osaka Gas Chemicals Co., Ltd. (mesh 4 to 6), and the aldehyde removal test was conducted.

EXAMPLES 3 TO 12

In Examples 3 to 12, deodorant compositions were prepared in the same manner as in Example 1 except that the loading solution used in Example 1 was changed to the polysiloxane solutions shown in Table 2 (prepared in Synthetic Examples 2 to 11), and the aldehyde removal test was conducted.

COMPARATIVE EXAMPLES 1 TO 2

In Comparative Examples 1 to 2, aldehyde removal rates were measured in the same manner as in Example 1 except that the deodorant composition was changed to activated carbons (Comparative Example 1: Shirasagi KL, and Comparative Example 2: Shirasagi G2x) without dropwise addition of a polysiloxane solution followed by drying.

COMPARATIVE EXAMPLE 3

In Comparative Example 3, a deodorant composition was prepared in the same manner as in Example 1 except that the loading solution used in Example 1 was changed to the polysiloxane solution prepared in Synthetic Example 12, and the aldehyde removal test was conducted.

The results of Examples 1 to 12 and Comparative Examples 1 to 3 are shown in Table 2. It is clear that the deodorant compositions according to the disclosure showed higher aldehyde removal rates than activated carbons alone and a deodorant composition comprising a polysiloxane having no aminooxyalkyl groups (Comparative Example 3).

[Table 2]

| | Raw materials* of loading solution [wt%] | | | Molecular weight | Activated carbon mesh | Loading [w/w] | Removal rate [%] |
|---|---|---|---|---|---|---|---|
| | Si-1 | Silane compound | Additive | Mw | | | |
| Ex. 1 | 7.8 | - | - | 1100 | 10-60 | 100% | 91 |
| Ex. 2 | 7.8 | - | - | 1100 | 4-6 | 100% | 83 |
| Ex. 3 | 20 | - | - | 990 | 10-60 | 100% | 98 |
| Ex. 4 | 7.8 | - | - | 1300 | 10-60 | 100% | 99 |
| Ex. 5 | 7.8 | - | 1 mol/L hydrochloric acid 32.9 | 910 | 10-60 | 100% | 97 |
| Ex. 6 | 7.8 | - | 1 mol/L sulfuric acid 3.3 | 1700 | 10-60 | 100% | 92 |
| Ex. 7 | 7.8 | - | 14.6 mol/L phosphoric acid 0.2 | 1200 | 10-60 | 100% | 93 |
| Ex. 8 | 7.8 | Si-2, 0.8 | - | 920 | 10-60 | 100% | 95 |
| Ex. 9 | 7.8 | Si-3, 9.7 | - | 1600 | 10-60 | 100% | 93 |
| Ex. 10 | 7.8 | TEOS, 2.1 | - | 1200 | 10-60 | 100% | 95 |
| Ex. 11 | 7.8 | TEMS, 10 | - | 3900 | 10-60 | 100% | 99 |
| Ex. 12 | 7.8 | DEDMS, 13 | - | 2500 | 10-60 | 100% | 91 |

(continued)

| | Raw materials* of loading solution [wt%] | | | Molecular weight | Activated carbon mesh | Loading [w/w] | Removal rate [%] |
|---|---|---|---|---|---|---|---|
| | Si-1 | Silane compound | Additive | Mw | | | |
| Comp. Ex. 1 | - | | | | 10-60 | - | 42 |
| Comp. Ex. 2 | - | | | | 4-6 | - | 61 |
| Comp. Ex. 3 | - | Si-2, 7.3 | - | 770 | 10-60 | 100% | 44 |
| * The balance is water. | | | | | | | |

EXAMPLE 13

<Preparation of Deodorizing Filter>

2 g of the loading solution was diluted by a factor of 4 with 6 g of 50 vol% aqueous ethanol. 0.43 mL of the resulting diluted solution was added dropwise to a non-woven filter containing activated carbon (UF-APN, manufactured by UES Inc.) cut into a 5-cm square, and the filter was dried in a hot-air dryer (ADVANTEC DRJ433DA) at 60°C for 30 minutes to obtain a deodorizing filter comprising an aminooxyalkyl-containing polysiloxane. The polysiloxane loading relative to the activated carbon and the amount of the activated carbon in the filter were calculated from the following formula.

Loading [wt%] = (Amount of diluted solution added [mL] × Density of diluted solution [g/mL] ÷ Dilution factor) / (Nominal amount of activated carbon in filter [g/m$^2$] × 0.0025 [m$^2$]) × 100

Amount of activated carbon in filter [wt%] = Nominal amount of activated carbon in filter [g/m2] / Mass of filter [g/m$^2$] × 100

<Acetaldehyde Removal Test>

The deodorizing filter was sealed in a 10 L Tedlar bag, and 5 L of nitrogen gas containing 10 ppm acetaldehyde was injected. After 1 hour of standing at room temperature, the gas in the Tedlar bag was passed through a 2,4-dinitrophenylhydrazine (DNPH) cartridge column (Precep-C DNPH, manufactured by FUJIFILM Wako Pure Chemical Corporation) to adsorb the aldehyde in the gas on it. The DNPH-aldehyde condensate was eluted from the column with acetonitrile. The eluate was analyzed with a liquid chromatograph (LC-2030C Plus, manufactured by Shimadzu Corporation) to determine the concentration of the acetaldehyde. The acetaldehyde removal rate was calculated from the following formula.

Acetaldehyde removal rate [%] = [(Initial acetaldehyde concentration - Remaining acetaldehyde concentration) / Initial acetaldehyde concentration] × 100

EXAMPLES 14 TO 15

In Examples 14 to 15, deodorant compositions were prepared in the same manner as in Example 13 except that the deodorizing filter used in Example 13 was changed to a non-woven filter containing activated carbon UF-APM, manufactured by UES Inc. in Example 14 or to a urethan filter containing activated carbon UF-PU3, manufactured by UES Inc. in Example 15, and their acetaldehyde removal rates were measured.

COMPARATIVE EXAMPLES 4 TO 6

The acetaldehyde removal test was conducted in the same manner as in Example 13 except that as the deodorant composition, a non-woven filter containing activated carbon UF-APN, a non-woven filter containing activated carbon UF-APM and a urethan filter containing activated carbon UF-PU3 were used in Comparative Examples 4, 5 and 6,

respectively, without dropwise addition of the loading solution followed by drying.

The results of Examples 13 to 15 and Comparative Examples 4 to 6 are shown in Table 3. It is clear from Table 3 that the deodorant compositions according to the disclosure improve the aldehyde removal rate of activated carbon held on a filter.

[Table 3]

| | Composition of diluted loading solution [wt%] | | Loading [w/w] | Filter substrate | Amount of activated carbon in filter [w/w] | Removal rate [%] |
|---|---|---|---|---|---|---|
| | Siloxane solution prepared in Synthetic Ex. 1 | 50% aq. ethanol | | | | |
| Ex. 13 | 25 | 75 | 19% | Non-woven fabric | 36% | 92 |
| Ex. 14 | 25 | 75 | 33% | Non-woven fabric | 83% | 94 |
| Ex. 15 | 25 | 75 | 40% | Urethan | 41% | 69 |
| Comp. Ex. 4 | - | | | Non-woven fabric | 36% | 73 |
| Comp. Ex. 5 | - | | | Non-woven fabric | 83% | 51 |
| Comp. Ex. 6 | - | | | Urethan | 41% | 32 |

EXAMPLE 16

<Preparation of Deodorizing Filter>

2 g of the loading solution was diluted by a factor of 4 with 1 mol/L hydrochloric acid (0.66 g, 0.66 mmol) and water (5.34 g). 0.39 mL of the resulting diluted loading solution was added dropwise to a non-woven filter containing activated carbon (UF-APN, manufactured by UES Inc.) cut into a 5-cm square, and the filter was dried in a hot-air dryer (ADVANTEC DRJ433DA) at 60°C for 30 minutes to obtain a deodorizing filter comprising an aminooxyalkyl-containing polysiloxane. The aminooxyalkyl-containing polysiloxane loading relative to the activated carbon and the amount of the activated carbon in the filter were calculated from the following formula.

Loading [wt%] = (Amount of diluted solution added [mL] × Density of diluted solution [g/mL] ÷ Dilution factor) / (Nominal amount of activated carbon in filter [g/$m^2$] × 0.0025 [$m^2$]) × 100

Amount of activated carbon in filter [wt%] = Nominal amount of activated carbon in filter [g/m2] / Mass of filter [g/$m^2$] × 100

<Acetaldehyde Removal Test>

The deodorizing filter was sealed in a 10 L Tedlar bag, and 5 L of nitrogen gas containing 10 ppm acetaldehyde was injected. After 1 hour of standing at room temperature, the gas in the Tedlar bag was passed through a 2,4-dinitrophenylhydrazine (DNPH) cartridge column (Precep-C DNPH, manufactured by FUJIFILM Wako Pure Chemical Corporation) to adsorb the aldehyde in the gas on it. The DNPH-aldehyde condensate was eluted from the column with acetonitrile. The eluate was analyzed with a liquid chromatograph (LC-2030C Plus, manufactured by Shimadzu Corporation) to determine the concentration of the acetaldehyde. The acetaldehyde removal rate was calculated from the following formula.

Acetaldehyde removal rate [%] = [(Initial acetaldehyde concentration - Remaining acetaldehyde concentration) / Initial acetaldehyde concentration] × 100

EXAMPLES 17 TO 23

In Examples 17 to 23, deodorant compositions were prepared in the same manner as in Example 16 except that the diluted solution used in Example 16 was changed to 4-fold diluted polysiloxane solutions shown in Table 4, and their acetaldehyde removal rates were measured.

The results of Examples 16 to 23 are shown in Table 4. It is clear from Table 4 that the deodorant compositions

according to the disclosure show high aldehyde removal rates even when they contain an acid, a base or a surfactant.

[Table 4]

| | Composition of diluted loading solution* [wt%] | | Loading [w/w] | Amount of activated carbon in filter [w/w] | Removal rate [%] |
|---|---|---|---|---|---|
| | Siloxane solution prepared in Synthetic Ex. 1 | Additive | | | |
| Ex.16 | 25 | 1 mol/L hydrochloric acid, 8.3 | 33% | 83% | 91 |
| Ex.17 | 25 | 1 mol/L sulfuric acid, 8.3 | 33% | 83% | 94 |
| Ex.18 | 25 | 14.6 mol/L phosphoric acid, 0.6 | 33% | 83% | 98 |
| Ex.19 | 25 | Sodium trifluoromethanesulfonic acid, 1.4 | 33% | 83% | 95 |
| Ex.20 | 25 | Barbituric acid, 1.1 | 33% | 83% | 96 |
| Ex.21 | 25 | Sodium carbonate, 0.9 | 33% | 83% | 93 |
| Ex.22 | 25 | Tris(hydroxymethyl) aminomethane, 1.0 | 33% | 83% | 97 |
| Ex.23 | 25 | Sodium dodecylbenzenesulfonate, 2.9 | 33% | 83% | 99 |
| * The balance is water. | | | | | |

EXAMPLE 24

<Preparation of Deodorant Composition>

0.3 g of the loading solution was added dropwise to 0.3 g of activated carbon (Shirasagi C2c, manufactured by Osaka Gas Chemicals Co., Ltd., mesh 20 to 48), and the activated carbon was dried in a hot-air dryer (ADVANTEC DRJ433DA) at 60°C for 6 hours to obtain a deodorant composition comprising an aminooxyalkyl-containing polysiloxane.

<Acetaldehyde Removal Test>

0.25 g of the deodorant composition was sealed in a 10 L Tedlar bag, and 5 L of nitrogen gas containing 1 ppm acetaldehyde was injected. After 4 hours of standing at room temperature, the gas in the Tedlar bag was passed through a 2,4-dinitrophenylhydrazine (DNPH) cartridge column (Precep-C DNPH, manufactured by FUJIFILM Wako Pure Chemical Corporation) to adsorb the aldehyde in the gas on it. The DNPH-aldehyde condensate was eluted from the column with acetonitrile. The eluate was analyzed with a liquid chromatograph (LC-2030C Plus, manufactured by Shimadzu Corporation) to determine the concentration of the acetaldehyde. The acetaldehyde removal rate was calculated from the following formula.

Acetaldehyde removal rate [%] = [(Initial acetaldehyde concentration - Remaining acetaldehyde concentration) / Initial acetaldehyde concentration] × 100

Then, the deodorant composition was withdrawn from the Tedlar bag and stored in air at room temperature for 3 months. 3 months later, the deodorant composition was sealed in a Tedlar bag again and subjected to the acetaldehyde removal test.

EXAMPLE 25

In Example 25, a deodorant composition was prepared in the same manner as in Example 24 except that the loading solution used in Example 24 was changed to the polysiloxane solution prepared in Synthetic Example 9, and the aldehyde removal test was conducted.

COMPARATIVE EXAMPLE 7

In Comparative Example 7, a deodorant composition was prepared in the same manner as in Example 24 except that the loading solution used in Example 24 was changed to 3% aqueous solution of the following formula (2) (referred to

as aminooxyacetic acid in Table 5), and the aldehyde removal test was conducted.

The results of Examples 24 to 25 and Comparative Example 7 are shown in Table 5.

[Table 5]

| | Raw materials of loading solution [wt%] | | | Activated carbon mesh | Loading [w/w] | Removal rate [%] | |
|---|---|---|---|---|---|---|---|
| | Si-1 | TEOS | Water | | | Immediately after loading | 3 months later |
| Ex. 24 | 7.8 | - | 92.2 | 20-48 | 100% | 97 | 96 |
| Ex. 25 | 7.8 | 2.1 | 90.1 | 20-48 | 100% | 95 | 97 |
| Comp. Ex. 7 | 3% Aqueous aminooxyacetic acid | | | 20-48 | 100% | 80 | 54 |

It is clear from Table 5 that the deodorant compositions according to the disclosure can remove aldehydes efficiently even after long storage in air, compared to the deodorant composition comprising a conventional aldehyde scavenger.

EXAMPLE 26

<Preparation of Deodorizing Filter>

1 g of the loading solution was diluted by a factor of 17 with 16 g of water. Two non-woven filters containing activated carbon (UF-APM, manufactured by UES Inc.) cut into a 5-cm square were immersed in the resulting diluted solution and squeezed through a press machine (IMC-6600, manufactured by Imoto Machinery Co., Ltd.). The filters were dried in a hot-air dryer (ADVANTEC DRJ433DA) under different drying conditions, one at 60°C for 30 minutes, and the other at 130°C for 1 hour, to obtain two deodorizing filters. The polysiloxane loading relative to the activated carbon and the amount of the activated carbon in the filter were calculated from the following formula.

Loading [wt%] = (Mass of squeezed filter [g] - Mass of filter before immersion [g]) ÷ Dilution factor) / (Nominal amount of activated carbon in filter [g/m$^2$] × 0.0025 [m$^2$]) × 100

Amount of activated carbon in filter [wt%] = Nominal amount of activated carbon in filter [g/m$^2$] / Mass of filter [g/m$^2$] × 100

<Acetaldehyde Removal Test>

Each of the deodorizing filters was sealed in a 10 L Tedlar bag, and 5 L of nitrogen gas containing 10 ppm acetaldehyde was injected. After 1 hour of standing at room temperature, the gas in each Tedlar bag was passed through a 2,4-dinitrophenylhydrazine (DNPH) cartridge column (Precep-C DNPH, manufactured by FUJIFILM Wako Pure Chemical Corporation) to adsorb the aldehyde in the gas on it. The DNPH-aldehyde condensate was eluted from the columns with acetonitrile. The eluates were analyzed with a liquid chromatograph (LC-2030C Plus, manufactured by Shimadzu Corporation) to determine the concentration of the acetaldehyde. The acetaldehyde removal rate was calculated from the following formula.

Acetaldehyde removal rate [%] = [(Initial acetaldehyde concentration - Remaining acetaldehyde concentration) / Initial acetaldehyde concentration] × 100

EXAMPLE 27

In Example 27, a deodorant composition was prepared in the same manner as in Example 26 except that the

loading solution used in Example 26 was changed to the polysiloxane solution prepared in Synthetic Example 9, and the aldehyde removal test was conducted.

COMPARATIVE EXAMPLE 8

In Comparative Example 8, one of two non-woven filters containing activated carbon (UF-APM manufactured by UES Inc.) cut into a 5-cm square was dried at 60°C for 30 minutes, and the other was dried at 130°C for 1 hour. The aldehyde removal test was conducted in the same manner as in Example 26 except that as the deodorizing filters, these filters were used.

COMPARATIVE EXAMPLE 9

In Comparative Example 9, deodorizing filters were prepared in the same manner as in Example 26 except that the loading solution used in Example 26 was changed to a 3% aqueous solution of the compound represented by the formula (2), and the acetaldehyde removal test was conducted.

COMPARATIVE EXAMPLE 10

In Comparative Example 10, deodorizing filters were prepared in the same manner as in Example 26 except that the loading solution used in Example 26 was changed to a 3% aqueous solution of adipic dihydrazide (manufactured by Tokyo Chemical Industry Co., Ltd.), and the acetaldehyde removal test was conducted.

COMPARATIVE EXAMPLE 11

In Comparative Example 11, deodorizing filters were prepared in the same manner as in Example 26 except that the loading solution used in Example 26 was changed to a 3% aqueous solution of 4-amino-1,2,4-triazole (manufactured by Tokyo Chemical Industry Co., Ltd.), and the acetaldehyde removal test was conducted.

The results of Examples 26 to 27 and Comparative Examples 8 to 11 are shown in Table 6. It is clear from Table 6 that the deodorizing filters of the present invention can remove aldehydes efficiently even after high-temperature drying, as compared to the deodorizing filter comprising a conventional aldehyde scavenger.

[Table 6]

| | Raw materials of loading solution [wt%] | | | Loading [w/w] | Amount of activated carbon in filter [w/w] | Removal rate | |
|---|---|---|---|---|---|---|---|
| | Si-1 | TEOS | Water | | | After 30 minutes of drying at 60°C | After 1 hour of drying at 130°C |
| Ex. 26 | 7.8 | - | 92.2 | 8% | 83% | 84 | 97 |
| Ex. 27 | 7.8 | 2.1 | 90.1 | 8% | 83% | 87 | 92 |
| Comp. Ex. 8 | - | | | | 83% | 51 | 56 |
| Comp. Ex. 9 | 3% Aqueous aminooxyacetic acid | | | 8% | 83% | 95 | 52 |
| Comp. Ex. 10 | 3% Aqueous adipic dihydrazide | | | 8% | 83% | 96 | 75 |
| Comp. Ex. 11 | 3% Aqueous 4-amino-1,2,4-triazole | | | 8% | 83% | 89 | 74 |

EXAMPLE 28

<Preparation of Deodorizing Filter>

1 g of the polysiloxane solution (hereinafter referred to as the loading solution) prepared in Synthetic Example 1 was diluted by a factor of 17 with 16 g of water. Three non-woven filters containing activated carbon (UF-APM, manufactured by UES Inc.) cut into a 6-cm square were immersed in the resulting diluted solution, squeezed through a press machine (IMC-6600, manufactured by Imoto Machinery Co., Ltd.) and dried in a hot-air dryer (ADVANTEC DRJ433DA) at 60°C for 30 minutes to obtain three deodorizing filters comprising an aminooxyalkyl-containing polysiloxane. The deodorant loading relative to the activated carbon was calculated from the following formula.

Loading [wt%] = (Mass of squeezed filter [g] - Mass of filter before immersion [g]) × deodorant concentration in diluted solution [%]) / (Nominal amount of activated carbon in filter [g/m$^2$] × 0.0025 [m$^2$]) × 100

<Acetaldehyde Removal Test>

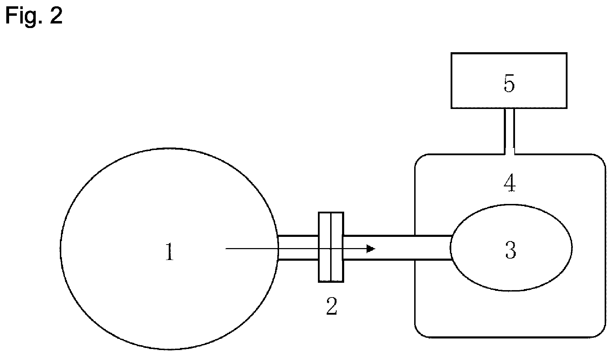

One of the filters was clamped between flanges on a stainless steel pipe (crosssectional flow area 13 cm$^2$) at the right angle to the flow path so as to completely cover the flow path cross-section. The pipe was connected to a 100 L Tedlar bag at one end, and was connected to a sealed vessel containing a 5 L Tedlar bag at the other end. The sealed vessel was connected to a suction pump (MP-Σ300NII, manufactured by SIBATA SCIENTIFIC TECHNOLOGY LTD.) so that as the gas in the sealed vessel is sucked by the pump, the gas in the 100 L Tedlar bag flows through the filter and is collected into the 5 L Tedlar bag. Fig. 2 shows the schematic operation of the tester.

100 L of nitrogen gas containing 4 ppm acetaldehyde was injected into the 100 L Tedlar bag, and the suction pump was operated at a flow rate of 0.5 L/min (0.007 m/sec) until 3 L of an aldehyde-containing gas was collected. The aldehyde-containing gas was passed through a 2,4-dinitrophenylhydrazine (DNPH) cartridge column (Precep-C DNPH, manufactured by FUJIFILM Wako Pure Chemical Corporation) to adsorb the aldehyde in the gas on it. The DNPH-aldehyde condensate was eluted from the column with acetonitrile. The eluate was analyzed with a liquid chromatograph (LC-2030C Plus, manufactured by Shimadzu Corporation) to determine the concentration of the acetaldehyde. The acetaldehyde removal rate was calculated from the following formula.

Acetaldehyde removal rate [%] = [(Initial acetaldehyde concentration - Remaining acetaldehyde concentration) / Initial acetaldehyde concentration] × 100

Then, the suction pump was changed to LV-40BW manufactured by SIBATA SCIENTIFIC TECHNOLOGY LTD. The above-mentioned procedure was followed by using the other two deodorant compositions except that the suction pump operated set at a flow rate of 14 L/min (0.18 m/sec) for one of the deodorant composition and at a flow rate of 40 L/min (0.53 m/sec) for the other, to collect aldehyde-containing gases, and the aldehyde removal rates were calculated.

EXAMPLE 29

In Example 29, deodorizing filters were prepared in the same manner as in Example 28 except that the loading solution used in Example 28 was changed to the polysiloxane solution prepared in Synthetic Example 9, and the acetaldehyde removal test was conducted.

COMPARATIVE EXAMPLE 12

In Comparative Example 12, the aldehyde removal test was conducted in the same manner as in Example 28 except that as the deodorizing filter, non-woven filters containing activated carbon (UF-APM, manufactured by UES Inc.) cut into a 6-cm square were used without loading, and the aldehyde removal test was conducted.

COMPARATIVE EXAMPLE 13

In Comparative Example 13, deodorizing filters were prepared in the same manner as in Example 28 except that the loading solution used in Example 28 was changed to a 40% aqueous solution of 4-amino-1,2,4-triazole (manufactured by Tokyo Chemical Industry Co., Ltd.), and the acetaldehyde removal test was conducted.

The results of Examples 28 to 29 and Comparative Examples 12 to 13 are shown in Table 7. It is clear from Table 7 that the aldehyde removal rates of the deodorizing filters of the present invention did not decrease as much as the aldehyde removal rate of the deodorizing filter comprising a conventional aldehyde scavenger when the flow rate of acetaldehyde gas increased.

[Table 7]

| | Raw materials of loading solution [wt%] | | | Loading [w/w] | Removal rate [%] | | |
|---|---|---|---|---|---|---|---|
| | Si-1 | TEOS | Water | | Flow rate 0.007 [m/sec] | Flow rate 0.18 [m/sec] | Flow rate 0.53 [m/sec] |
| Ex. 28 | 7.8 | - | 92.2 | 8% | 99 | 75 | 59 |
| Ex. 29 | 7.8 | 2.1 | 90.1 | 8% | 98 | 78 | 56 |

(continued)

| | Raw materials of loading solution [wt%] | | | Loading [w/w] | Removal rate [%] | | |
|---|---|---|---|---|---|---|---|
| | Si-1 | TEOS | Water | | Flow rate 0.007 [m/sec] | Flow rate 0.18 [m/sec] | Flow rate 0.53 [m/sec] |
| Comp. Ex. 12 | - | | | | 48 | 21 | 16 |
| Comp. Ex. 13 | 40% Aqueous 4-amino-1,2,4-triazole | | | 8% | 93 | 30 | 17 |

The present invention has been described in detail with reference to specific embodiments. However, it is apparent to those skilled in the art that various changes and modifications are possible without departing from the nature and the scope of the present invention.

The entire disclosure of Japanese Patent Application No. 2023-171201 filed on October 2, 2023 including specifications, claims, drawings and summaries is incorporated herein by reference in its entirety.

REFERENCE SYMBOLS

1: 100 L Tedlar bag

2: Flanges equipped with filter

3: 5 L Tedlar bag

4: 10 L Sealed vessel

5: Suction pump

**Claims**

**1.** A deodorant composition comprising activated carbon and an aminooxyalkyl-containing polysiloxane having a structural unit represented by the following formula (1) which is loaded on the activated carbon.

(1)

[wherein each R is independently a $C_{1-4}$ alkyl group which may be substituted with a primary amino group at any position, a $C_{1-4}$ alkoxy group or a hydroxy group, each Q is independently at least one group selected from the group consisting of $-CH_2-$, $-CH_2CH_2-$and $-CH_2-Ra-CH_2-$ wherein Ra is a $C_{1-18}$ alkylene group in which two or more interspaced $-CH_2-$ groups may each be independently replaced by -O-, -(C=O)O-, - O(C=O)-, -O(C=O)-O-, -C(=O)-NH-, -NH-(C=O)-, -CH=CH- or -C=C-, and in which a hydrogen atom in the alkylene group may be substituted with a $C_{1-3}$ alkyl group, and n is a real number of from 0 to 100.]

**2.** The deodorant composition according to Claim 1, wherein in the aminooxyalkyl-containing polysiloxane represented by the formula (1), each R is independently a methyl group, a 3-aminopropyl group, a methoxy group, an ethoxy group, a propyloxy group, an isopropyloxy group or a hydroxy group.

**3.** The deodorant composition according to Claim 1, wherein in the aminooxyalkyl-containing polysiloxane represented by the formula (1), n is 0.

**4.** The deodorant composition according to Claim 1, wherein the amount of the aminooxyalkyl-containing polysiloxane represented by the formula (1) is from 0.1 to 100 wt% of the activated carbon.

**5.** A deodorizing filter comprising the deodorant composition as defined in any one of Claims 1 to 4.

**6.** The deodorizing filter according to Claim 5, wherein the content of the deodorant composition is from 1 to 90 wt% of the deodorizing filter.

**7.** A method for removing an aldehyde, which comprises bringing the deodorant composition as defined in any one of Claims 1 to 4 into contact with a gas containing an aldehyde.

**8.** A method for removing an aldehyde, which comprises bringing the deodorizing filter as defined in Claim 5 or 6 into contact with a gas containing an aldehyde.

Fig. 1

Intens.
+MS, 2.1-2.6min #126-156, Background Subtracted
1243.9654
659.2856
815.1861
1543.9495
1250
1000
750
500
250
0
500
750
1000
1250
1500
1750
2000
2250
2500
2750
m/z

Fig. 2

5
4
1
3
2

**INTERNATIONAL SEARCH REPORT**

International application No. **PCT/JP2024/033478**

**A. CLASSIFICATION OF SUBJECT MATTER**

***A61L 9/014***(2006.01)i; ***A61L 9/01***(2006.01)i; ***B01J 20/22***(2006.01)i; ***C08G 77/26***(2006.01)i
FI: A61L9/014; A61L9/01 H; C08G77/26; B01J20/22 A

According to International Patent Classification (IPC) or to both national classification and IPC

**B. FIELDS SEARCHED**

Minimum documentation searched (classification system followed by classification symbols)

A61L9/014; A61L9/01; B01J20/22; C08G77/26

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2022/202984 A1 (TOSOH CORPORATION) 29 September 2022 (2022-09-29) paragraphs [0017], [0018], [0034], [0085], [0086], [0102], [0103], table 1, claims | 1-4, 7<br>5-6, 8 |
| X<br>Y | JP 2023-84176 A (TOSOH CORPORATION) 19 June 2023 (2023-06-19) claims, paragraphs [0014]-[0017], [0049], [0050], examples | 1-4, 7<br>5-6, 8 |
| X<br>A | JP 2023-84172 A (TOSOH CORPORATION) 19 June 2023 (2023-06-19) claims, paragraphs [0013]-[0016], [0041], [0042], examples | 1-4, 7<br>5-6, 8 |
| Y<br>A | JP 2018-114463 A (OSAKA GAS CHEMICALS CO., LTD.) 26 July 2018 (2018-07-26) claims, paragraphs [0029]-[0036], [0045] | 5-6, 8<br>1-4, 7 |
| A | JP 4-180834 A (NIPPONDENSO CO., LTD.) 29 June 1992 (1992-06-29) | 1-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 November 2024** | **03 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/033478**

**C. DOCUMENTS CONSIDERED TO BE RELEVANT**

| Category* | Citation of document, with indication, where appropriate, of the relevant passages | Relevant to claim No. |
|---|---|---|
| A | JP 2009-247419 A (KAO CORPORATION) 29 October 2009 (2009-10-29) | 1-8 |
| A | JP 2013-75070 A (SUMIKA ENVIRO-SCIENCE CO., LTD.) 25 April 2013 (2013-04-25) | 1-8 |

Form PCT/ISA/210 (second sheet) (July 2022)

**INTERNATIONAL SEARCH REPORT**
**Information on patent family members**

International application No.
**PCT/JP2024/033478**

| Patent document cited in search report | Publication date (day/month/year) | Patent family member(s) | Publication date (day/month/year) |
|---|---|---|---|
| WO 2022/202984 A1 | 29 September 2022 | US 2024/0198314 A1 paragraphs [0012], [0013], [0045], [0092], [0093], [0109], [0110], table 1, claims<br>EP 4316533 A1<br>CN 117120104 A | |
| JP 2023-84176 A | 19 June 2023 | (Family: none) | |
| JP 2023-84172 A | 19 June 2023 | (Family: none) | |
| JP 2018-114463 A | 26 July 2018 | (Family: none) | |
| JP 4-180834 A | 29 June 1992 | US 5238899 A<br>EP 486015 A1 | |
| JP 2009-247419 A | 29 October 2009 | (Family: none) | |
| JP 2013-75070 A | 25 April 2013 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H11299878 A
- JP 2000300652 A
- JP 2007215818 A
- JP 2018108360 A
- JP 2023171201 A